# EUROPEAN PATENT SPECIFICATION

(11) **EP 3 960 101 B1**
(45) Date of publication and mention of the grant of the patent: **31.07.2024**
(21) Application number: 20924938.2
(22) Date of filing: 22.04.2020
(51) Int. Cl.: A61B 17/32, A61C 3/03, A61C 1/05, A61B 17/3207

(54) **VIBRATION TYPE REMOVAL APPARATUS**
VORRICHTUNG ZUM ENTFERNEN VON SCHWINGUNGEN
APPAREIL D'ÉLIMINATION À VIBRATIONS

(30) Priority: 10.03.2020 JP 2020040674
(43) Date of publication of application: 02.03.2022
(73) Proprietor: Micron Machinery Co., Ltd., Yamagata-shi, Yamagata 990-2303 (JP)
(72) Inventor: KOBAYASHI, Satoshi, Yamagata-shi, Yamagata 990-2303 (JP); MINAGAWA, Yoshihiro, Yamagata-shi, Yamagata 990-2303 (JP); SUZUKI, Hiroki, Yamagata-shi, Yamagata 990-2303 (JP); SAKAKIBARA, Makoto, Yamagata-shi, Yamagata 990-2303 (JP)
(74) Representative: Cabinet Nony
(86) International application number: PCT/JP2020/017411
(87) International publication number: WO 2021/181714

(56) References cited:
- EP-A1- 1 880 689
- CN-A- 103 431 894
- JP-A- 2002 143 177
- JP-A- 2009 539 500
- JP-A- 2017 504 400
- JP-A- 2019 088 452
- JP-A- 2019 088 452
- JP-A- H02 286 149
- JP-A- S61 159 952
- JP-U- 3 207 943
- US-A- 4 832 683
- US-A1- 2008 183 109
- US-A1- 2013 123 774
- US-A1- 2014 163 595
- US-A1- 2016 051 378
- US-A1- 2017 258 486
- US-B1- 6 632 233
- US-B2- 10 555 748

## Description

### Technical Field

The present invention relates to a vibration type removal apparatus that peels and removes a hard tissue from a soft tissue using a tool attached to its distal end portion.

### Background Art

A vibration type cutting apparatus that comprises a vibration device and cuts an object to be removed by vibrating a tool attached to a distal end portion of the vibration device has been known (e.g., Patent Literature 1).

In the vibration type cutting apparatus described in Patent Literature 1 and a general rotation type cutting apparatus, vibration of a tool by a vibration device is controlled to burst oscillation in which stop and vibration are repeated, to improve operability.

In the vibration type cutting apparatus described in Patent Literature 1, when surgery for removing a hard tissue adhering to a soft tissue is performed, the hard tissue is cut using the tool from the side of a surface opposite to its surface adhering to the soft tissue, and the hard tissue is cut until the thickness thereof is a small thickness, e.g., a thickness of approximately one sheet. The thinned hard tissue is carefully manually removed by a handpiece such as a curette.

US 4 832 683 A discloses a vibration type removal apparatus that can peel and remove a hard tissue from a soft tissue.

US 2014/163595 A1 discloses an ultrasonic tool for scraping soft tissue from bone having a plurality of recesses along a width direction.

### Citation List

### Patent Literature

Patent Literature 1: Japanese Patent Laid-Open No. 2019-088452

### Summary of Invention

### Technical Problem

However, when a hard tissue is cut until it becomes thin, an unintended deviation of a distal end of a tool easily leads to a damage to a soft tissue because a force is applied to the tool in a direction in which the soft tissue exists. Particularly when a rotary tool is used, frictional heat generated on a contact surface with a thin hard tissue produces the possibility of damaging a soft tissue closest to the hard tissue. Thus, surgery (cutting work) needs to be carefully performed, so that a surgery time period is lengthened.

The present invention has been made in view of the foregoing points, and is directed to providing a vibration type removal apparatus capable of efficiently peeling and removing a hard tissue from a soft tissue.

### Solution to Problem

[1] A vibration type removal apparatus according to the present invention is a vibration type removal apparatus as claimed in claim 1, the vibration type removal apparatus comprising a housing, a vibration device arranged in the housing, a tool provided in a distal end portion of the vibration device, protruding from the housing, and insertable between the hard tissue and the soft tissue, a control device that controls driving of the vibration device to vibrate the tool in an axial direction of the vibration device, and a cooling device that cools the tool, in which the control device controls the vibration of the tool by the vibration device to burst oscillation in which stop and vibration are repeated.

According to the present invention, the tool provided in the distal end portion of the vibration device and protruding from the housing is insertable between the hard tissue and the soft tissue. Thus, when the tool is inserted between the hard tissue as an object to be removed and the soft tissue and is vibrated with a force applied in a direction away from the soft tissue, the hard tissue can be peeled and removed from the soft tissue by cutting a surface, which adheres to the soft tissue, of the hard tissue. As a result, the hard tissue can be removed in a shorter time period and more stably than when the hard tissue is cut until it becomes thin.

The tool is cooled by the cooling device, and the vibration of the tool is further controlled to burst oscillation in which stop and vibration are repeated. Thus, the tool can be more prevented from generating heat so that heat to be applied to the soft tissue and a peripheral tissue can be more suppressed than when the tool is always vibrated.

[2] A first surface, which opposes the soft tissue, of the tool has a plurality of recesses formed thereon.

This configuration makes it possible to more efficiently cool the tool than when the first surface, which opposes the soft tissue, of the tool is a flat surface having no unevenness.

[3] A second surface, which opposes the hard tissue, of the tool is preferably formed in a circular arc shape, and also a planar shape or a concave shape.

This configuration makes it easier to insert the tool between the hard tissue and the soft tissue than when the second surface, which opposes the hard tissue, of the tool has a projection shape.

[4] The cooling device is preferably composed of a cooling liquid supply device that supplies a cooling liquid, and the housing is preferably provided with a flow path that causes the cooling liquid supplied from the cooling liquid supply device to flow toward a distal end side of the tool. A distal end of the flow path is preferably provided to spray the cooling liquid toward a distal end portion of the tool.

This configuration makes it possible to cool the tool with cooling liquid supplied from the cooling liquid supply device and flowing through the flow path, and thus makes it possible to more efficiently cool the tool.

### Brief Description of Drawings

FIG. 1 is an explanatory view related to a configuration of a vibration type removal apparatus according to an embodiment of the present disclosure.

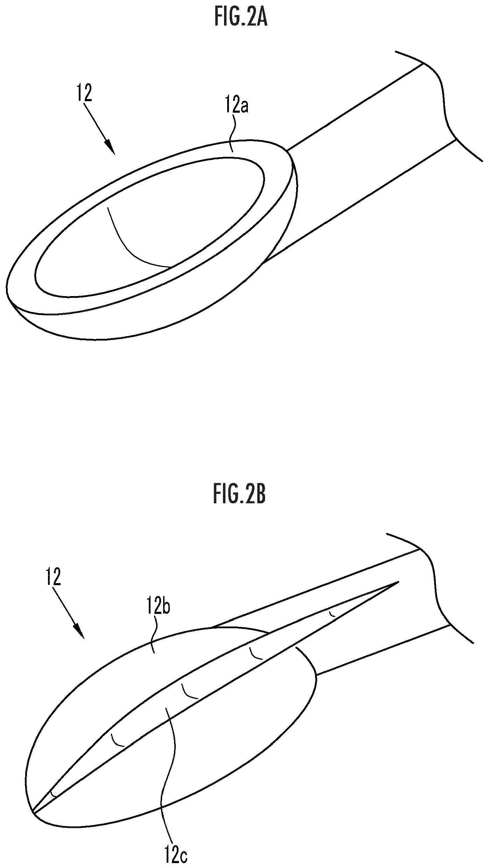

FIG. 2A is a perspective view illustrating a tool.
FIG. 2B is a perspective view illustrating the tool.
FIG. 3A is a schematic view illustrating the tool, a hard tissue, and a soft tissue.
FIG. 3B is a schematic view illustrating a state where the tool is inserted between the hard tissue and the soft tissue.
FIG. 3C is a schematic view illustrating a state where a hard tissue is peeled and removed using the tool.

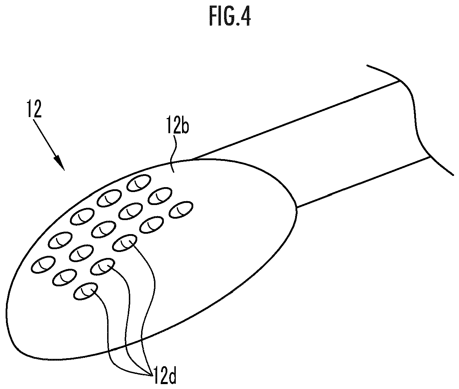

FIG. 4 is a perspective view illustrating a tool having a plurality of recesses formed therein, in accordance with the present claimed invention.

### Description of Embodiment

An embodiment of the present invention will be described below with reference to the drawings.

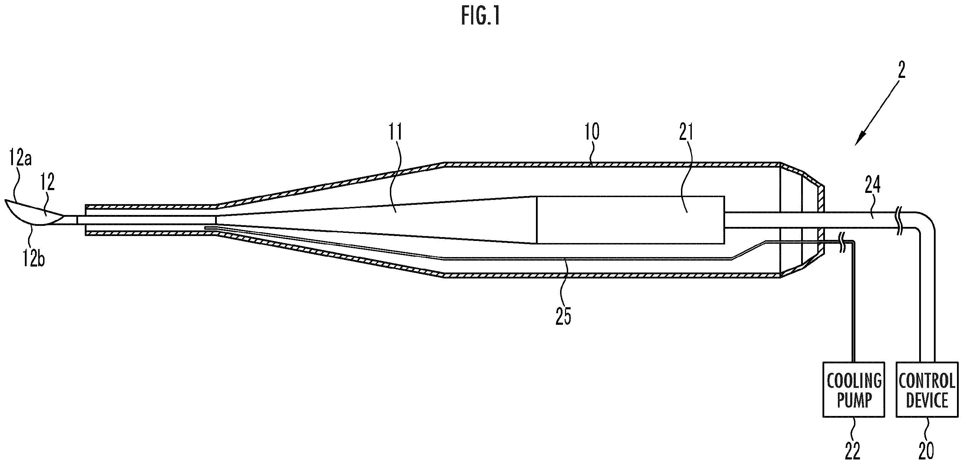

As illustrated in FIG. 1, a vibration type removal apparatus 2 according to an embodiment of the present invention comprises a housing 10 having a substantially cylindrical shape, a holding member 11, a tool 12, a control device 20, a vibration device 21, and a cooling pump 22, which constitute a handpiece.

The housing 10 is designed to a size small enough for a normal person to hold it with one hand. Although a handpiece as a high-frequency vibration type cutting apparatus is constituted by components such as the housing 10 and the holding member 11 at least a part of which is arranged in an inner space of the housing 10, the respective types and specifications of the components may be appropriately selected from the viewpoint of making the handpiece lightweight for simplicity of handling.

The holding member 11 has its rear end portion attached to the vibration device 21, and is supported to be movable in its axial direction on the housing 10 via a support section (not illustrate) fixed to an inner sidewall of the housing 10.

The holding member 11 has a function as a horn for increasing an amplitude. The tool 12 is detachably attached to a distal end portion of the holding member 11.

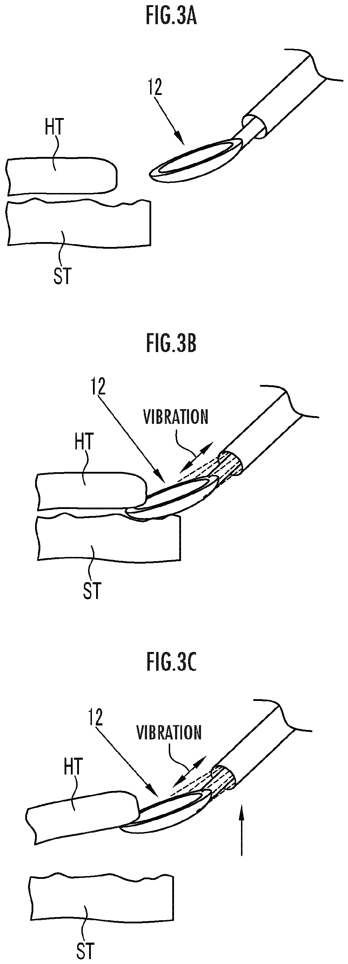

The tool 12 may be easily insertable between a hard tissue HT and a soft tissue ST (see FIG. 3B), and examples of the type of the tool 12 include a curette, a chisel, a scalpel, a file, a long type, and a short type. A blade having a linear shape or a circular arc shape at its distal end or a tool having any shape such as a substantially columnar shape, a spoon shape, or a bent or curved rod shape is adopted as the tool 12.

In the present embodiment, the tool 12 is composed of a curette, a distal end portion of the tool 12 has a hemispherical shape, a first surface 12a, which is obtained by cutting a sphere in half, of the tool 12 is formed in a circular arc shape and a concave shape, and a groove 12c extending toward the distal end portion from a proximal end portion on the holding member 11 side of the tool 12 is formed on a second surface 12b as a hemispherical portion of the tool 12.

The vibration device 21 is attached to an attachment section (not illustrated) in the housing 10 and is composed of a piezoelectric element arranged in the inner space of the housing 10 so that the holding member 11 is vibrated or driven to reciprocate in the axial direction.

The vibration device 21 and the holding member 11 are arranged such that their respective axes are common and arranged away from each other in their respective axial directions. Accordingly, spaces respectively occupied by the vibration device 21 and the holding member 11 in the inner space of the housing 10, and thus the housing 10 can be made more compact in a direction perpendicular to the axes than when the axes are arranged parallel to and away from each other or arranged nonparallel to each other. As a result, a vibration type removal apparatus can be configured as a handpiece ease of handling and operability of which are improved.

A force of the vibration device 21 is directly transmitted to the holding member 11 without a transmission mechanism being used. Accordingly, a lubricant such as grease to generally be used for the transmission mechanism is not required. Therefore, when the vibration type removal apparatus 2 as medical equipment is subjected to sterilization treatment with high-pressure steam, a situation where contamination of the medical equipment derived from the existence of the lubricant occurs is avoided.

The control device 20 is connected to the vibration device 21 via a cable 24 attached to a rear end portion of the housing 10, to control supply of power to the vibration device 21.

The control device 20 controls an operation of the vibration device 21. The control device 20 is composed of a microcomputer or a processor. The control device 20, together with a substrate on which it is mounted, may be arranged in the inner space of the housing 10.

The control device 20 performs control such that a vibration frequency f2 in an axial direction of the tool 12 via the holding member 11 by the vibration device 21 falls within a range of 20 to 60 [kHz]. The vibration frequency f2 is more preferably controlled to 25 to 45 [kHz].

Further, the control device 20 controls the vibration of the tool 12 by the vibration device 21 to burst oscillation in which stop and vibration are repeated, and an overall burst frequency f1 of the tool 12 in which a burst period during which the tool 12 is vibrated and a stop period during which the tool 12 is stopped are combined into one cycle is controlled depending on the cooling efficiency of the tool 12.

With the vibration type removal apparatus 2 having the above-described configuration, the holding member 11 is driven to reciprocate in the axial direction, whereby an object is cut using the tool 12 provided in the distal end portion of the holding member 11.

The control device 20 controls the vibration of the tool 12 by the vibration device 21 to burst oscillation in which vibration and stop are repeated.

Specifically, the control device 20 controls the burst frequency f1 of the tool 12 to a range of 1 to 300 [Hz] as a low frequency at which an operator can recognize a cycle of a burst period and a stop period, and controls a duty ratio d1 obtained by dividing a pulse width t1 of the burst frequency by a pulse period T1 to a range of 5 to 50 [%].

The cooling pump 22 sends out (supplies) cooling water sent from a cooling water supply source (not illustrated), and the driving thereof is controlled by the control device 20.

A flow path 25 that causes cooling water supplied from the cooling pump 22 to flow is attached to the rear end portion of the housing 10. The flow path 25 extends to a distal end portion from the rear end portion of the housing 10.

### [Peeling and Removal]

A case where the hard tissue HT as an object to be removed is peeled and removed using the vibration type removal apparatus 2 will be described.

As illustrated in FIG. 3A, the operator who performs peeling and removal (surgery) first attaches the tool 12 to the distal end portion of the holding member 11.

Then, the operator causes the control device 20 to drive the cooling pump 22, as illustrated in FIG. 3B. As a result, cooling water supplied from the cooling pump 22 passes through the flow path 25 in the housing 10, and is sprayed toward the tool 12 from a distal end of the flow path 25. In this state, the operator inserts the tool 12 between the hard tissue HT and the soft tissue ST. In FIG. 3B and FIG. 3C, cooling water is indicated by a dotted line for simplification.

As illustrated in FIG. 3C, the operator causes the control device 20 to operate the vibration device 21, to drive the holding member 11 to reciprocate in the axial direction. As a result, the tool 12 provided in the distal end portion of the holding member 11 is driven to reciprocate (vibrated) in the axial direction. At this time, the operator applies a force in a direction away from the soft tissue ST (in an upward direction in FIG. 3C).

Due to the vibration of the tool 12, the hard tissue HT is cut from the side of an adhering surface of the soft tissue ST and the hard tissue HT, and the hard tissue HT is peeled from the soft tissue ST. As a result, the hard tissue HT can be removed in a shorter time period and more stably than when the hard tissue HT is cut until it becomes thin toward the soft tissue ST.

In the present embodiment, the hard tissue HT is a bone, an ossified cell, or the like of a predetermined portion of a human body, and the soft tissue ST is a nerve, a blood vessel, or the like adjacent to the hard tissue HT. Accordingly, the safety of the soft tissue ST needs to be ensured in the surgery.

In the present embodiment, the control device 20 controls the vibration of the tool 12 by the vibration device 21 to burst oscillation in which vibration and stop are repeated. As a result, the tool 12 can be more prevented from generating heat so that the safety of the soft tissue ST can be more ensured than when the tool 12 is always vibrated or a tool which is rotated.

The tool 12 is cooled with cooling water. Thus, the tool 12 can be further prevented from generating heat.

Further, the groove 12c can be 12e is formed on the second surface 12b, which contacts the soft tissue ST, of the tool 12, and cooling water is also sent to the groove 12c. As a result, a cooling effect with cooling water can be enhanced.

Although the tool 12 is cooled with cooling water in the above-described embodiment, for example, the cooling is not limited to the cooling with cooling water as long as the tool 12 can be cooled. For example, cooling gas may be sprayed onto the tool 12.

The groove 12c of the tool 12 may be extended toward a rear end portion of the tool 12 so that cooling water is directly poured into the groove 12c.

Although the first surface 12a of the tool 12 is formed in a circular arc shape and a concave shape in the above-described embodiment, the first surface 12a may be formed in a circular arc shape and a planar shape.

Further, although the groove 12c extending toward the distal end side from the proximal end (rear end) side of the tool 12 is formed on the second surface 12b of the tool 12 in the above-described embodiment, the shape of the groove 12c is appropriately changeable. For example, a spiral groove may be formed. Further, a plurality of grooves may be formed.

A plurality of recesses 12d as illustrated in Fig, 4 are formed in accordance with the claimed invention. In this case, a cooling effect is also enhanced.

Further, a shape of the flow path 25 is appropriately changeable. For example, the flow path 25 may be formed in such a spiral shape as to surround the holding member 11. A flow path for causing cooling water to flow may be formed in the tool 12.

### Reference Signs List

2...vibration type removal apparatus, 10...housing, 11...holding member, 12...tool, 20...control device, 21...vibration device, 22...cooling pump, HT...hard tissue, ST...soft tissue

## Claims

1. A vibration type removal apparatus (2) that can peel and remove a hard tissue (HT) from a soft tissue (ST), the vibration type removal apparatus (2) comprising:
a housing (10);
a vibration device (21) arranged in the housing (10);
a tool (12) provided in a distal end portion of the vibration device (21), protruding from the housing (10), and insertable between the hard tissue (HT) and the soft tissue (ST);
a control device (20) that controls driving of the vibration device (21) to vibrate the tool (12) in an axial direction of the vibration device (21); and
a cooling device (22) that cools the tool (12), **characterised in that**:
a first surface (12a), which opposes the soft tissue (ST), of the tool (12) has a plurality of recesses (12d) formed thereon, along each of the axial direction of the vibration device (21) and a width direction of the tool (12), and
the control device (20) controls the vibration of the tool (12) by the vibration device (21) to burst oscillation in which stop and vibration are repeated.

2. The vibration type removal apparatus (2) according to claim 1, wherein
a second surface (12b), which opposes the hard tissue (HT), of the tool (12) is formed in a circular arc shape, and also a planar shape or a concave shape.

3. The vibration type removal apparatus (2) according to claim 1, wherein
the cooling device (22) is composed of a cooling liquid supply device that supplies a cooling liquid, and
the housing (10) is provided with a flow path (25) that causes a cooling liquid supplied from the cooling liquid supply device to flow toward a distal end side of the tool (12).

## Patentansprüche

1. Vorrichtung (2) zum Entfernen durch Schwingungen, die ein hartes Gewebe (HT) von einem weichen Gewebe (ST) schälen und entfernen kann, wobei die Vorrichtung (2) zum Entfernen durch Schwingungen umfasst:
ein Gehäuse (10);
eine Schwingungsvorrichtung (21), die in dem Gehäuse (10) angeordnet ist;
ein Instrument (12), das in einem distalen Endabschnitt der Schwingungsvorrichtung (21) vorgesehen ist, aus dem Gehäuse (10) herausragt und zwischen das harte Gewebe (HT) und das weiche Gewebe (ST) einführbar ist;
eine Steuervorrichtung (20), die den Antrieb der Schwingungsvorrichtung (21) steuert, um das Instrument (12) in einer axialen Richtung der Schwingungsvorrichtung (21) in Schwingung zu versetzen; und
eine Kühlvorrichtung (22), die das Instrument (12) kühlt,
**dadurch gekennzeichnet, dass**:
eine erste Oberfläche (12a) des Instruments (12), die dem weichen Gewebe (ST) gegenüberliegt, eine Vielzahl von darauf ausgebildeten Vertiefungen (12d) entlang jeder der axialen Richtung der Schwingungsvorrichtung (21) und einer Breitenrichtung des Instruments (12) aufweist, und die Steuervorrichtung (20) die Schwingung des Instruments (12) durch die Schwingungsvorrichtung (21) so steuert, dass die Oszillation, bei der sich Stillstand und Schwingung wiederholen, ausbricht.

2. Vorrichtung zum Entfernen durch Schwingungen (2) gemäß Anspruch 1, wobei
eine zweite, dem harten Gewebe (HT) gegenüberliegende Fläche (12b) des Instruments (12) sowohl kreisbogenförmig als auch plan oder konkav ausgebildet ist.

3. Vorrichtung zum Entfernen durch Schwingungen (2) gemäß Anspruch 1, wobei
die Kühlvorrichtung (22) aus einer Kühlflüssigkeitszufuhrvorrichtung besteht, die eine Kühlflüssigkeit zuführt, und
das Gehäuse (10) mit einem Strömungsweg (25) versehen ist, der bewirkt, dass eine aus der Kühlflüssigkeitszufuhrvorrichtung zugeführte Kühlflüssigkeit zu einer distalen Endseite des Instruments (12) fließt.

## Revendications

1. Appareil d'ablation de type vibrant (2) apte à décoller et à réaliser l'ablation d'un tissu dur (HT) vis-à-vis d'un tissu mou (ST), l'appareil d'ablation de type vibrant (2) comprenant :
un boîtier (10) ;
un dispositif vibrant (21) agencé dans le boîtier (10) ;
un outil (12) placé dans une partie d'extrémité distale du dispositif vibrant (21), faisant saillie du boîtier (10), et insérable entre le tissu dur (HT) et le tissu mou (ST) ;
un dispositif de commande (20) qui commande l'excitation du dispositif vibrant (21) pour faire vibrer l'outil (12) dans une direction axiale du dispositif vibrant (21) ; et
un dispositif de refroidissement (22) qui refroidit l'outil (12), **caractérisé en ce que** :
une première surface (12a), opposée au tissu mou (ST), de l'outil (12) est dotée d'une pluralité d'évidements (12d), le long à la fois de la direction axiale du dispositif vibrant (21) et d'une direction en largeur de l'outil (12), et
le dispositif de commande (20) commande la vibration de l'outil (12) par le dispositif vibrant (21) selon une oscillation en salves à arrêt et vibration répétés.

2. Appareil d'ablation de type vibrant (2) selon la revendication 1, dans lequel
une deuxième surface (12b), opposée au tissu dur (HT), de l'outil (12) est pourvue d'une forme en arc de cercle, et également d'une forme plane ou d'une forme concave.

3. Appareil d'ablation de type vibrant (2) selon la revendication 1, dans lequel
le dispositif de refroidissement (22) se compose d'un dispositif d'apport de liquide de refroidissement qui apporte un liquide de refroidissement, et
le boîtier (10) est pourvu d'un chemin d'écoulement (25) qui provoque l'écoulement d'un liquide de refroidissement apporté par le dispositif d'apport de liquide de refroidissement en direction du côté d'une extrémité distale de l'outil (12).
